# EUROPEAN PATENT SPECIFICATION

(11) **EP 4 216 941 B1**
(45) Date of publication and mention of the grant of the patent: **11.11.2026**
(21) Application number: 21873130.5
(22) Date of filing: 28.07.2021
(51) Int. Cl.: A61K 31/64, A61K 31/18, A61K 31/19, A61K 31/198, A61K 31/337, A61K 31/397, A61K 31/401, A61K 31/445, A61K 31/495, A61K 31/4965, A61P 3/04, A61P 43/00

(54) **PARA-TOLUENESULPHONAMIDE FOR USE IN THE TREATMENT OF LIPOMA**
PARA-TOLUENESULPHONAMID ZUR VERWENDUNG BEI DER BEHANDLUNG VON LIPOMEN
PARA-TOLUÈNESULPHONAMIDE POUR UTILISATION DANS LE TRAITEMENT DU LIPOME

(30) Priority: 24.09.2020 US 202017030902
(43) Date of publication of application: 02.08.2023
(73) Proprietor: GONGWIN BIOPHARM CO., LTD. (TAIWAN), Taipei City 10491 (TW)
(72) Inventor: WU, Shun-chi, Taipei City, 10449 (TW); LIN, Mao-Yuan, Taipei City, 10449 (TW); CHEN, Di-Rong, Taipei City, 10449 (TW); YANG, Chuan-Ching, Taipei City, 10449 (TW); ZHONG, Nan-Shan, Guangzhou Guangdong, 510120 (CN); TU, Chi-Chiang, Taipei City, 10449 (TW)
(74) Representative: Icosa
(86) International application number: PCT/US2021/043434
(87) International publication number: WO 2022/066277

(56) References cited:
- EP-A1- 3 243 510
- WO-A1-2005/063247
- WO-A1-2018/029150
- KR-A- 20030 095 697
- BALARAMNAVAR VISHAL M.; SRIVASTAVA ROHIT; RAHUJA NEHA; GUPTA SWATI; RAWAT ARUN K.; VARSHNEY SALIL; CHANDASANA HARDIK; CHHONKER YAS: "Identification of novel PTP1B inhibitors by pharmacophore based virtual screening, scaffold hopping and docking", EUROPEAN JOURNAL OF MEDICINAL CHEMISTRY, ELSEVIER, AMSTERDAM, NL, vol. 87, 2 October 2014 (2014-10-02), AMSTERDAM, NL , pages 578 - 594, XP029019107, ISSN: 0223-5234, DOI: 10.1016/j.ejmech.2014.09.097
- EMIMMAL MAIDA ENGELS SELVARAJ, SANKAR VEINTRAMUTHU: "Synthesis and Pancreatic Lipase Inhibitory Activity of Phenacyl Esters of N-Aroyl Amino Acids", CURRENT ENZYME INHIBITION, BENTHAM SCIENCE PUBLISHERS LTD, HILVERSUM, NL, vol. 15, no. 2, 19 October 2019 (2019-10-19), NL , pages 133 - 143, XP055915674, ISSN: 1573-4080, DOI: 10.2174/1573408015666190920092114

## Description

### REFERENCE TO RELATED APPLICATIONS

This application claims the benefit of the filing date of U.S. Patent Application No. 17/030,902, filed on September 24, 2020.

### BACKGROUND OF THE INVENTION

### 1. Technical Field

The present disclosure relates a composition comprising para-toluene sulfonamide and a pharmaceutically acceptable carrier for use in preventing or treating lipoma.

### 2. Description of Related Art

Lipoma is a benign tumor of the skin. It is formed by excessive proliferation of mature adipose cells and usually has a soft and round appearance. The outermost layer thereof is covered with a thin fibrous membrane that contains fiber bundles. In some cases, lipomas accompany proliferation of different proportions of blood vessels, muscle, cartilage, bone marrow cells, sweat glands, thymus, thyroid gland, etc. The edges of the lipoma are clear and can be moved. Generally, lipomas are found in the soft tissue layer under the skin, such as limbs or torso, but not in other internal organs, such as the gastrointestinal wall, abdominal cavity, or fascia. Lipomas usually grow in the soft tissue layer and are less likely to have malignant lesions. However, if lipomas grow in the internal organs or in the posterior abdominal cavity, they would suddenly enlarge or the edges thereof would become unclear, because other lesions may cause malignant changes of the lipoma or benign lipoma may turn into malignant tumor.

As well know, solitary lipoma refers to only one lipoma, whereas multiple lipoma refers to more than two lipomas. People having lipoma usually would not feel painful or itchy; nevertheless, if lipomas grow in areas with dense blood vessels or nerves, it may result in obvious tenderness due to the natural contraction of blood vessels or compression of surrounding nerves and tissues. Clinically, the small lipoma may have only one centimeter in diameter, while the large one may exceed beyond ten centimeters.

Lipomas are usually caused by excessive fat proliferation, such that people with obesity are prone to suffer from lipomas. However, the occurrence of lipoma is not necessarily related to obesity or diet (e.g., greasy diet and excessive fat intake). Due to recombination and mutations in chromosomes, fat cells in certain parts of the body may be abnormally proliferated. Some lipomas are highly related to genetics, such as familial multiple lipomas. In some other patients, the adipose tissue may be abnormally stimulated after a trauma is healed, causing the generation of lipomas at the original injured site.

Since lipomas are usually benign, no urgency for treatment would be required, unless it causes local pain that affects the function of body or the quality of life. If lipomas are suspected of being malignant, local pain would be inexhaustible, and the tumor would greatly affect the appearance and life that requires further medical treatment. Currently, lipomas are usually treated by surgery. If the lipoma is too large, a small hole can be punctured, and then the lipoma can be smashed and further squeezed out. It can also be treated by liposuction. However, in the treatment of lipomas, there are still problems such as unsuitability or inability to carry out at the growth site of lipoma, surgical side effects, postoperative pain or related risks that may lead to abandonment of the treatment.

Up to now, there is still no product containing pharmaceutical compositions related to the treatment of lipomas. Therefore, it is an unmet need to develop a pharmaceutical composition that can locally reduce fat in the body of a subj ect with lower side effects, better stability, and shorter recovery period. Given the high demands of patients suffering from obesity or lipoma, the development of a pharmaceutical composition for fat ablation or lipolysis that can break through the limitations of current technology would be an urgent issue to be resolved. EP 3 243 510 A1 discloses para-toluenesulfonamide for use in the treatment of cancer.

### SUMMARY OF THE INVENTION

The present disclosure provides a composition comprising para-toluene sulfonamide and a pharmaceutically acceptable carrier for use in preventing or treating lipoma in a subject in need thereof, wherein the composition is to be administered to the subject in an effective amount.

### BRIEF DESCRIPTIONS OF THE DRAWINGS

FIGs. 1A to 1F show H&E staining (FIGs. 1A, 1B, 1C and 1D) and Masson's trichrome staining (FIGs. 1E and 1F) of the adipose tissue of the rat injected with p-toluenesulfonamide (FIGs. 1A, 1C and 1E) or saline (FIGs. 1B, 1D and 1F). Scale bars represent 200 µm.
FIG. 2 shows the volume change of infiltrative benign lipoma in lesion (A) of the dog treated with the GWA101 drug.
FIG. 3 shows the volume change of infiltrative benign lipoma in lesion (B) of the dog treated with the GWA101 drug.

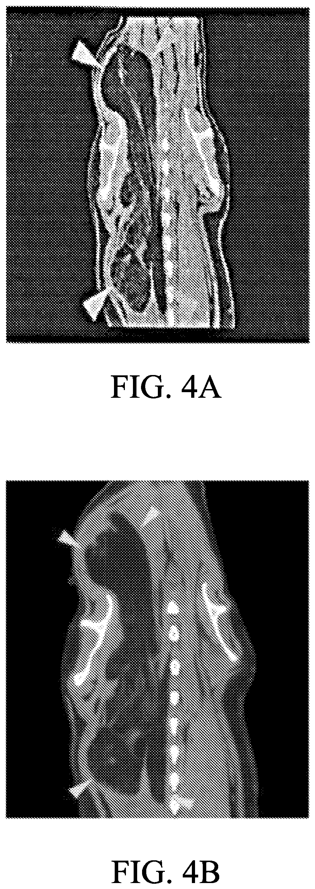

FIGs. 4A and 4B show the results of computed tomography scan for infiltrative benign lipoma in lesion (A) and lesion (B) of the dog. FIGs. 4A shows the result of computed tomography scan for infiltrative benign lipoma in lesion (A) and lesion (B) of the dog before the first administration of the GWA101 drug. FIG. 4B shows the result of computed tomography scan for infiltrative benign lipoma in lesion (A) and lesion (B) of the dog at conclusion visit after the 8th administration of the GWA101. Arrows indicates the changes of computed tomography scan on lesions (A) and (B) before and after the administration.
FIG. 5 shows the volume change of cystic benign lipoma in the lesion of the dog treated with the GWA101 drug.
FIGs. 6A and 6B show the results of computed tomography scan for cystic benign lipoma in the lesion of the dog. FIG. 6A shows the result of computed tomography scan for cystic benign lipoma in the lesion of the dog before the first administration of the GWA101 drug. FIG. 6B shows the result of computed tomography scan for cystic benign lipoma in the lesion of the dog at conclusion visit after the 7th administration of the GWA101. Arrows indicates the changes of computed tomography scan on the lesion before and after the administration.
FIG. 7 shows the appearance change of the skin on the lipoma lesion of the dog treated with the GWA101 drug.

### DETAILED DESCRIPTION OF THE INVENTION

The following examples are used to exemplify the present disclosure. A person of ordinary skill in the art can understand the other advantages of the present disclosure, based on the specification of the present disclosure. The present disclosure can also be implemented or applied as described in different examples. It is possible to modify and/or alter the above examples for carrying out this disclosure without contravening its scope for different aspects and applications.

It is noted that, as used in this specification, the singular forms "a," "an," and "the" include plural referents unless expressly and unequivocally limited to one referent. The term "or" is used interchangeably with the term "and/or" unless the context clearly indicates otherwise.

The present disclosure provides a composition comprising para-toluene sulfonamide and a pharmaceutically acceptable carrier for use in preventing or treating lipoma in a subject in need thereof, wherein the composition is to be administered to the subject in an effective amount.

In one embodiment of the present disclosure, the pharmaceutically acceptable carrier may be a filler, a binder, a preservative, a disintegrating agent, a lubricant, a suspending agent, a wetting agent, a flavoring agent, a thickening agent, an acid, a biocompatible solvent, a surfactant, a complexation agent, or any combination thereof.

In an embodiment of the present disclosure, the pharmaceutically acceptable carrier may be polyethylene glycol (PEG), alkylene glycol, propylene glycol, sebacic acid, dimethyl sulfoxide (DMSO), ethanol, or any combination thereof. The examples of the alkylene glycol include, but are not limited to, 2-ethyl-1,3-hexandiol and propanediol. The example of the PEG includes, but is not limited to, PEG-400.

In an embodiment of the present disclosure, the para-toluene sulfonamide is in an amount of 1% to 60% of the composition by weight. For example, an amount of the *para-*toluene sulfonamide in the composition has a lower limit chosen from 1%, 5%, 10%, 15%, 20%, and 25% of the composition by weight, and an upper limit chosen from 60%, 55%, 50%, 45%, 40%, and 35% of the composition by weight.

In an embodiment of the present disclosure, the pharmaceutically acceptable carrier is in an amount of 25% to 99% of the composition by weight. For example, an amount of the pharmaceutically acceptable carrier in the composition has a lower limit chosen from 25%, 30%, 35%, and 40% of the composition by weight, and an upper limit chosen from 99%, 95%, 90%, 80%, 70%, and 60% of the composition by weight.

In an embodiment of the present disclosure, the pharmaceutically acceptable carrier is chosen from at least one of 10% to 40% by weight of PEG, 5% to 10% by weight of propylene glycol, 1% to 5% by weight of sebacic acid, 0% to 15% by weight of *p-*Toluenesulfonic acid 10% to 20% by weight of 2-ethyl-1,3-hexanediol, 0% to 10% by weight of DMSO and 0% to 20% by weight of ethanol.

In an embodiment of the present disclosure, the composition may be formulated into a form suitable for parenteral administration, injection, continuous perfusion, sublingual administration, subcutaneous administration, topical administration, or oral administration. For example, the composition may be, but is not limited to, a formulation to injection, dry powder, a tablet, an oral liquid, a wafer, a film, a lozenge, a capsule, a granule, a pill, a gel, a lotion, an ointment, an emulsifier, a paste, or a cream.

In an embodiment of the present disclosure, the subject may be a mammal, such as a mouse, a rat, a dog, a primate, or a human.

In an embodiment of the present disclosure, the composition may be administered to the subject intratumorally, intravenously, subcutaneously, intradermally, intrathecally, intraperitoneally, intramuscularly, topically, orally, sublingually, or intrapleurally. In an embodiment of the present disclosure, the composition may be administered locally or topically to the fat deposits, tumor, muscle, intramuscular space, subcutaneous space, orbital space, skin, lesion, or mucous membrane of the subject. In an embodiment, the method of the present disclosure effectively ablate fat in the subject without causing severe side effects such as fibrosis, pain, local redness that brought about by traditional lipolysis injections.

In an embodiment of the present disclosure, the method comprises injecting the composition into an injection site of the subject. In an embodiment of the present disclosure, the injection site is a site of fat deposition or an intratumoral site.

In an embodiment of the present disclosure, the para-toluene sulfonamide in the composition may be administered to the subject in a therapeutically effective amount of from about 100 mg to about 26400 mg, such as 300 mg to 7000 mg or 3300 mg to 26400 mg.

In an embodiment of the present disclosure, the composition may be administered to the subject 1 to 4 times per week or 1 to 3 times per month.

In an embodiment of the present disclosure, the composition may be administered to the subject for a 1- to 4-week treatment period or 1- to 6-month treatment period.

As used herein, the term "lipoma" refers to a benign tumor composed of fat cells in the fatty tissue. Lipomas can occur almost anywhere in the body of the subject, but often occurs on the neck, arms, torso, thighs, or armpits of the subject. Types of lipoma include, but are not limited to, infiltrative lipoma, cystic benign lipoma, superficial subcutaneous lipoma, angiolipoma, intramuscular lipoma, angiolipoleiomyoma, neural fibrolipoma, benign lipoblastoma, pleomorphic lipoma, chondroid lipoma, spindle-cell lipoma, intradermal spindle cell lipoma, lipoma of tendon sheath, nerves, or synovium, and hibernoma. Although infiltrative lipoma belongs to benign tumor, it is very locally invasive and has a high tendency to recur after surgery. Malignant transformation of lipoma into liposarcoma is unusual; however, the malignant transformation has been found in the bone lipoma and the kidney lipoma.

In an embodiment of the present disclosure, the pharmaceutical composition can be used to treat lipoma by triggering the fat ablation or lipolysis in the body of the subject. In an embodiment of the present disclosure, the injection dosage for the fat ablation is 0.4 mL to 10 mL (about 132 mg to 3300 mg ofp-toluenesulfonamide).

In an embodiment of the present disclosure, the pharmaceutical composition can be directly injected into the lipoma or liposarcoma or injected into the area surrounding the lipoma or liposarcoma. In an embodiment of the present disclosure, the dosage can be proportionally increased or decreased according to the therapeutic situation. In an embodiment of the present disclosure, the administration of the pharmaceutical composition can occur 1 to 4 times a day, 1 to 4 times a week, or 1 to 3 times a month. In an embodiment of the present disclosure, the administration may continue until the lipoma or liposarcoma shrinks 5%, 10%, 15%, 20%, 25%, 30%, 35%, 40%, 45%, 50%, 55%, 60%, 65%, 70%, 75%, 80%, 85%, 90%, or 95% (by volume or weight), or until the lipoma or liposarcoma is fully eliminated.

The following are embodiments further demonstrating the efficacy of the current disclosure.

### EXAMPLES

The present disclosure is further described by means of the following examples.

### Preparation Example

### Pharmaceutical composition of benzenesulfonamides (GWA-101):

| | |
|---|---|
| *p*-Toluenesulfonamide | 1%-60% |
| PEG-400 | 10%-40% |
| 1,2-Propylene glycol | 5%-10% |
| Sebacic acid | 1%-5% |
| *p*-Toluenesulfonic acid | 0%-15% |
| 2-Ethyl-1,3-hexanediol | 10%-20% |
| Dimethyl sulfoxide | 0-10% |
| Ethanol | 0-20% |

Preparation of the composition of the present disclosure includes the process of: adding and mixing the solvents and adjuvants in a given ratio; heating the mixture to 80°C to 110°C with stirring to form a clear oily liquid; gradually adding the sulfa drug with stirring until completely dissolved; filtering and cooling the mixture to obtain the composition of the present disclosure in an oily liquid form (GWA-101).

The present disclosure also provides the use of GWA-101 as a medicament for promoting fat ablation or lipolysis in the body of the subject.

It is confirmed by animal tests that the pharmaceutical composition of the present disclosure can promote the ablation or lipolysis of normal adipose tissue in rats. It is further confirmed by clinical trials that the pharmaceutical composition of the present disclosure can improve the clinical symptoms in lipoma individuals with adipose hyperplasia to have better quality of life.

The efficacy of the fat ablation or lipolysis disclosed in the present disclosure was assessed by the following animal tests and clinical trials.

### EMBODIMENT 1

The rat model of fat ablation was established, and the pedicled inguinal fat pad of the hind limb of the rat was exposed by surgical incision. Further, 0.4 mL (approximately 132 mg of p-toluenesulfonamide) and 0.4 mL saline were injected directly into the right fat mass and the left fat mass of the rat, respectively. After completion of the injection into the adipose tissue, the incision was sutured. Four days after the treatment, the rat was sacrificed and sampled for interpretation on the pathological section. Hematoxylin and eosin (H&E) staining was used to determine the degree of necrosis and inflammation of the adipose tissue, and Masson's trichrome staining was used to determine the degree of fibrosis of the adipose tissue.

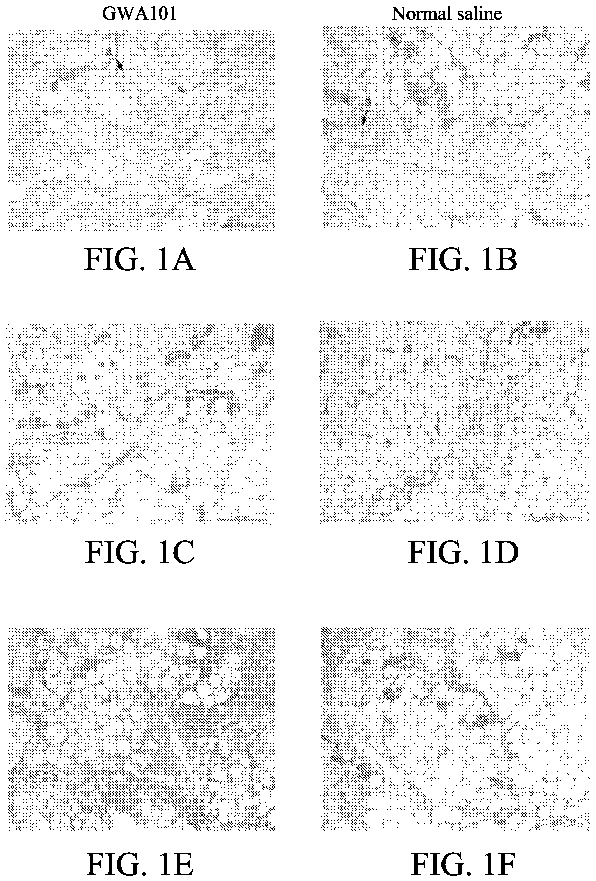

### Experimental results of the rat model for fat ablation (referring to Table 1 below and FIGs. 1A to 1F)

The results of the pathological section were entrusted to an impartial third-party veterinarian for single-blind interpretation. The results showed that as compared with the mild necrosis of the saline group, the GWA101 drug injection group caused obvious and large-scale necrosis of the adipose tissue (referring to the arrows shown in FIGs. 1A and 1B) and caused a higher infiltration of immune cells and slight fibrosis. From the results, it can be seen that the GWA101 drug injection can effectively ablate the local adipose tissue and induce local inflammation.

It is worth noting that although the phosphatidylcholine (PPC) lipolysis injections can effectively ablate fat in the past, it would easily cause tissue fibrosis (previously reported as an average between 25% to 75%), and the average degree of fibrosis after the GWA101 drug injection was less than 1%, which had the significant advantage of lower fibrotic side effects.

**Table 1**

| Organ | Histopathological finding | Pathological number | | | | | | | |
|---|---|---|---|---|---|---|---|---|---|
| | | GWA101 | | | | Normal Saline | | | |
| | | No. 1 | No. 2 | No. 3 | No. 4 | No. 1 | No. 2 | No. 3 | No. 4 |
| | Necrosis | 4^{*} | 4 | 4 | 2 | n | n | 1 | 2 |
| Adipose tissue | Inflammation, mononuclear cell and neutrophil | 2 | 4 | 2 | 2 | n | n | 2 | 2 |
| | fibrosis | 1 | 1 | 1 | 1 | n | n | n | 1 |

| | | | | | | | | | |
|---|---|---|---|---|---|---|---|---|---|
| n: No significant lesions * Degree of lesions stained with H&E was graded from 1 to 5 depending on severity: 1 = minimal (< 1% lesions); 2 = slight (1-25% lesions); 3 = moderate (26-50% lesions); 4 = moderate/severe (51-75% lesions); 5 = severe/high (76-100% lesions). | | | | | | | | | |

### EMBODIMENT 2

The case in the clinical trial was carried out at Wellcare Vet in Taipei, Taiwan in April 2020 below.
Subject animals: Dog
Name of affected dog: Big Sister
Dog breed: mixed
Gender: Female
Age: 15 years old
Weight: 16 kg to 17 kg
Diagnosis: infiltrative benign lipoma from the right shoulder scapula to the back
Disposal before treatment: No medical treatment
History of lipoma: infiltrative benign lipoma was diagnosed on March 16, 2020, which was characterized by slight to mild panniculitis and steatitis, and highly suspected of having infiltrative lipoma.

In this case, there were multifocal muscular or fibrotic tissue surrounding the type of adipose tissue. Infiltration of inflammatory cells was minimal to mild, indicating that it might be benign fat proliferation.

The inclusion criteria are: (A) the dog is greater than or equal to 1 year old; (B) the dog is diagnosed with lipoma by cytology or histopathology; (C) the trial veterinarian assesses that the dog is unsuitable for removal of the lipoma by surgery; (D) the dog has at least one measurable lesion that is larger than 1 cm in diameter; (E) the trial veterinarian assesses the life expectancy of the dog to exceed 3 months; and (F) the owner can understand and abide by the experimental procedure and is willing to sign an informed consent form.

The exclusion criteria are: (A) the dog has received systemic chemotherapy within 4 weeks before entering the trial; (B) the dog has received radiotherapy within 4 weeks before entering the trial; (C) the dog has underwent a major operation (for example, thoracotomy is not allowed, but the non-invasive operation, such as biopsy, is allowed) within 4 weeks before entering the trial; (D) the dog is treated by any other experimental drugs, biological formulations, medical materials, or other anti-tumor treatments (such as immunomodulators and radiotherapy) within 4 weeks before entering this trial or during the period of this trial experiment; (E) the dog has the following abnormal value of blood tests before entering the trial: a. hemoglobin < 6.0 g/dL; b. absolute neutrophil count (ANC) < 1,500/ *µ*L; c. albumin < 1.5 g/dL; d. total bilirubin < 2mg/dL; e. alanine aminotransferase (ALT) and aspartate aminotransferase (AST) > 5× upper normal limit (UNL); f. chronic kidney disease (CKD), the International Renal Interest Society (IRIS) > stage 3; (F) the dog suffers from any other serious diseases such as infection, uncontrolled diabetes, stage C of chronic degenerative valve disease (CDVD, one of the heart diseases), gastric ulcer, severe autoimmune disease, and the trial veterinarian restricts the animal from participating in this trial after assessment; (G) the dog is known or suspected of having allergic reactions to the ingredients contained in any *p*-toluenesulfonamide drugs; (H) the trial veterinarian diagnoses that the dog's lesion was blocked by important blood vessels, so it is difficult to perform intratumoral injection therein; (I) the dog is pregnant; and (J) the trial veterinarian determines that the dog is unsuitable for participating in this trial.

Those who met at least one of the following criteria should be withdrawn from clinical trials: (A) the informed consent form is withdrawn; (B) the dog receives the treatment prohibited by this trial; (C) after assessing any pathological characteristics, clinical adverse events, or any changes in the condition of the animal, the trial veterinarian determines that it is not the most advantageous situation to allow the dog to continue participating in the trial; (D) the dog is pregnant during the treatment period or is suspected of being pregnant by its owner or the trial veterinarian; (E) the dog has an adverse event of grade 3 or above according to international adverse events of oncology organization (Veterinary Cooperative Oncology Group - Common Terminology Criteria for Adverse Events, VCOG-CTCAE) and cannot return to grade 1 within 7 days after the adverse event, or the grade 3 or above adverse event still occurs after 2 dose reductions in the dog; (F) signs and symptoms of disease progression or deterioration (assessment of deterioration was based on Veterinary Cooperative Oncology Group - Response Evaluation Criteria in Solid Tumours v1.0 (VCOG-RECIST v1.0)); (G) death; (H) loss of follow-up tracking; and (I) violation of the plan.

### Treatment method:

The test pet was given intratumoral injections of 3.4, 1.8, 4.0, 4.5, 4.5, 4.5, 3.0, 3.0 mL of the GWA101 drug on Day 1, 6, 8, 13, 15, 20, 22, 28 (each time about 594 to 1485 mg of p-toluenesulfonamide). The injections were carried out in single, multi-point (4 to 5 points) intratumoral injections.

### Response assessment criteria:

The electronic vernier caliper measurement was performed before each administration, and the computed tomography (CT) scan was performed before the first administration and at the conclusion visit. Complete response (CR) means that a measurable or evaluable lesion disappears completely, and no new lesions appear for more than four weeks. Partial response (PR) means that a measurable or evaluable lesion shrinks by more than or equal to 30%, and no new lesions appear for more than four weeks. Stable disease (SD) means that a measurable or evaluable lesion shrinks by less than or equal to 30% or enlarges by less than or equal to 20%. Disease progression (PD) means that a measurable or evaluable lesion enlarges by more than or equal to 20%, or other lesions deteriorate, and new lesions appear.

### Safety assessment:

When conducting safety assessment during the trial period, the relevant researchers of the trial were responsible for defining and compiling the adverse events in the protocol (the method for assessment was referred to "Veterinary Cooperative Oncology Group - Common Terminology Criteria for Adverse Events (VCOG -CTCAE)."

The followings show the treatment results of lipoma in Embodiment 2.

### Interim efficacy (measured with the electronic vernier caliper):

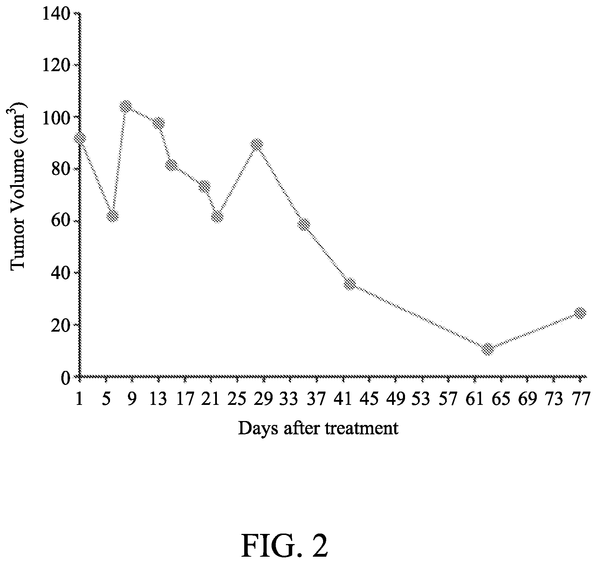

Regarding lesion (A), the measurement range thereof was located in the infiltrative lipoma of the right scapula of the dog. After 8 times administrations of the GWA101 drug, the volume of the lipoma changed. The volume of the lipoma was 91.80 cm³ before treatment, 89.31 cm³ before the 8th administration, and 24.41 cm³ at the assessment visit (7 weeks after the 8th administration). Comparing the volume of the lipoma before the first administration with that at the assessment visit, the measurable or assessable lesion shrinks by 73.4%, and the result was a partial response (PR) (referring to Table 2 below and FIG. 2).

**Table 2**

| | D1 | D6 | D8 | D13 | D15 | D20 | D22 | D28 | D35 | D42 | D63 | D77 |
|---|---|---|---|---|---|---|---|---|---|---|---|---|
| | T1 | T2 | T3 | T4 | T5 | T6 | T7 | T8 | RV | RV | RV | CV |
| a | 4.00 | 3.00 | 3.50 | 3.50 | 3.70 | 3.50 | 4.20 | 3.00 | 2.31 | 1.72 | 0.82 | 1.62 |
| b | 5.40 | 5.50 | 700 | 6.80 | 5.50 | 6.00 | 4.20 | 6.66 | 6.11 | 5.71 | 4.14 | 5.42 |
| c | 8.50 | 7.50 | 8.50 | 8.20 | 8.00 | 7.00 | 7.00 | 8.94 | 8.32 | 7.28 | 6.17 | 5.56 |
| Volume | 91.80 | 61.88 | 104.13 | 97.58 | 81.40 | 73.50 | 61.74 | 89.31 | 58.71 | 35.75 | 10.47 | 24.41 |

| | | | | | | | | | | | | |
|---|---|---|---|---|---|---|---|---|---|---|---|---|
| $Volume \left({cm}^{3}\right) = a \left(cm\right) \times b \left(cm\right) \times c \left(cm\right) \times 1 / 2$ D: Day; T: Treatment; RV: Random Visit; CV: Conclusion Visit | | | | | | | | | | | | |

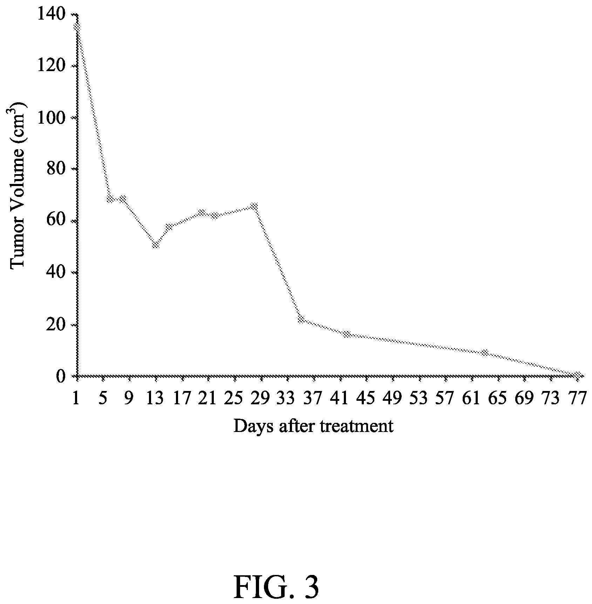

As to lesion (B), the measurement range thereof was located in the infiltrative lipoma on the back of the dog. After 8 times administrations of the GWA101 drug, the volume of the lipoma changed. The volume of the lipoma was 135.00 cm³ before treatment, 65.66 cm³ before the 8th administration, and 0.14 cm³ at the assessment visit (7 weeks after the 8th administration). Comparing the volume of the lipoma before the first administration with that at the assessment visit, the measurable or assessable lesion shrinks by 99.9%, and the result was a partial response (PR) (referring to Table 3 below and FIG. 3).

**Table 3**

| | D1 | D6 | D8 | D13 | D15 | D20 | D22 | D28 | D35 | D42 | D63 | D77 |
|---|---|---|---|---|---|---|---|---|---|---|---|---|
| | T1 | T2 | T3 | T4 | T5 | T6 | T7 | T8 | RV | RV | RV | RV |
| a | 4.50 | 2.60 | 3.50 | 2.20 | 3.50 | 3.00 | 4.20 | 3.00 | 1.25 | 0.88 | 0.66 | 0.01 |
| b | 7.50 | 750 | 6.50 | 6.30 | 5.50 | 6.00 | 4.20 | 5.70 | 5.52 | 5.94 | 5.06 | 5.15 |
| c | 8.00 | 7.00 | 6.00 | 7.30 | 6.00 | 7.00 | 7.00 | 7.68 | 6.31 | 6.22 | 5.39 | 5.56 |
| Volume | 135.00 | 68.25 | 68.25 | 50.59 | 57.75 | 63.00 | 61.74 | 65.66 | 21.77 | 16.26 | 9.00 | 0.14 |

| | | | | | | | | | | | | |
|---|---|---|---|---|---|---|---|---|---|---|---|---|
| $Volume \left({cm}^{3}\right) = a \left(cm\right) \times b \left(cm\right) \times c \left(cm\right) \times 1 / 2$ D: Day; T: Treatment; RV: Random Visit; CV: Conclusion Visit | | | | | | | | | | | | |

### Interim efficacy (computed tomography scan):

Regarding lesions (A+B), the measurement range thereof was located in the infiltrative lipomas of the right scapula and the back of the dog.

The results of computed tomography scan before the first administration are as follows: a huge, homogeneous, fat-reduced, cystic, and longitudinal lump with a central multifocal blurred area was found in the right neck area, starting from the horizontal (width: 5.40 cm, height: 8.50 cm, length: 22.20 cm) and between the right serratus muscle and cervical longitudinal muscle. The end of the lump infiltrated into the right longitudinal muscle and rhomboid muscle (FIG. 4A).

The results of computed tomography scan at conclusion visit are as follows: a large amount of even fat-reduced, low enhancement, cystic, longitudinal lump with a central multifocal blurred area was found in the right neck area and between the right serratus muscle and cervical longitudinal muscle (width: 5.26 cm, height: 8.20 cm, length: 21.50 cm). The lump infiltrated into the right longissimus and rhomboid muscles (FIG. 4B).

According to the above results of computed tomography scan, the volume of the infiltrative lipoma had been slightly reduced, and there is a large amount of fat loss inside the lipoma after the treatment.

### Adverse effects:

No common side effects such as pain, nausea, vomiting or local redness and inflammation had been observed. The functions of liver and kidney were stable and normal during and after the treatment.

### Conclusion of this embodiment:

The pharmaceutical composition of the present disclosure can reduce or ablate fat and thereby improve the quality of life and clinical symptoms of dogs with lipomas. Furthermore, no significant increase in adverse reactions has been observed in the clinical trials.

### EMBODIMENT 3

The clinical trial case was conducted at Wellcare Vet in Taipei, Taiwan in March 2020 as follows:
Subject animal: dog.
Dog breed: Labrador.
Gender: female.
Age: 14 years old.
Weight: 34 kg to 36 kg.
Diagnosis: Cystic benign lipoma located on the right scapula of the dog.
Disposal before treatment: No medical treatment.
History of lipoma: benign lipoma was diagnosed on March 10, 2020, which was characterized by being a subcutaneous adipose tissue.

In this case, the lipoma has myxomatous stroma and pulmonary spindle cells, and thus was suspected of being myxoma or myxosarcoma.

The inclusion criteria, the exclusion criteria, and the withdrawal criteria in this clinical trial were the same as those recited in Embodiment 2.

### Treatment method:

The dog was administered with 5.2 mL (about 1716 mg ofp-toluenesulfonamide each time) of the GWA101 drug by intratumoral injections on Days 1, 3, 5, 8, 10, 12, and 15. The injections were carried out in single, multi-point (4 to 5 points) intratumoral injections.

Response evaluation criteria and adverse effect criteria were the same as those recited in Embodiment 2. The followings show the treatment results of lipoma of Embodiment 3.

### Interim efficacy (measured with the electronic vernier caliper):

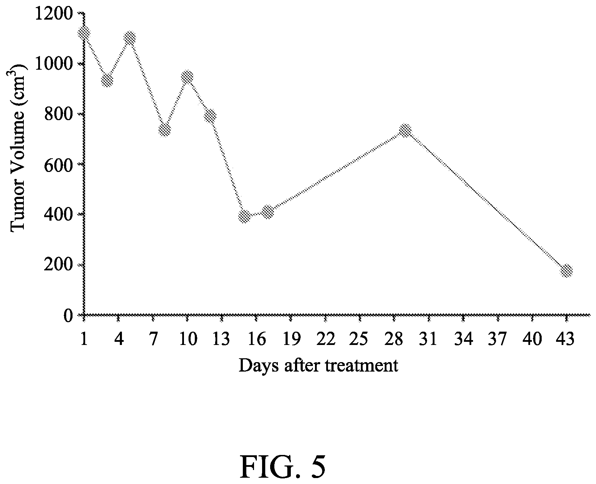

The measurement range of the lesion was a lipoma close to the scapula. After 7 times of administration of the GWA101 drug, the volume of the lipoma changed. The volume of the lipoma was 1,122 cm³ before treatment, 393 cm³ before the 7th administration, and 175 cm³ at the assessment visit (4 weeks after the 7th administration). Comparing the volume of the lipoma before the first administration with that at the assessment visit, the measurable or assessable lesion shrinks by 84.4%, and the result was a partial response (PR) (referring to Table 4 below and FIG. 5).

**Table 4**

| | D1 | D3 | D5 | D8 | D10 | D12 | D15 | D17 | D29 | D43 |
|---|---|---|---|---|---|---|---|---|---|---|
| | T1 | T2 | T3 | T4 | T5 | T6 | T7 | RV | RV | RV |
| a | 5.80 | 6.35 | 8.89 | 7.36 | 7.62 | 8.89 | 5.08 | 5.08 | 6.35 | 2.60 |
| b | 19.05 | 16.51 | 13.97 | 12.70 | 13.97 | 10.16 | 10.16 | 10.92 | 13.97 | 9.00 |
| c | 20.32 | 17.78 | 17.78 | 15.74 | 17.78 | 17.52 | 15.24 | 14.73 | 16.51 | 15.00 |
| Volume | 1122.58 | 932.01 | 1104.08 | 735.62 | 946.35 | 791.22 | 393.29 | 408.56 | 732.30 | 175.50 |

| | | | | | | | | | | |
|---|---|---|---|---|---|---|---|---|---|---|
| $Volume \left({cm}^{3}\right) = a \left(cm\right) \times b \left(cm\right) \times c \left(cm\right) \times 1 / 2$ D: Day; T: Treatment; RV: Random Visit; CV: Conclusion Visit | | | | | | | | | | |

### Interim efficacy (computed tomography scan):

The measurement range of the lesion was a lipoma close to the scapula of the dog.

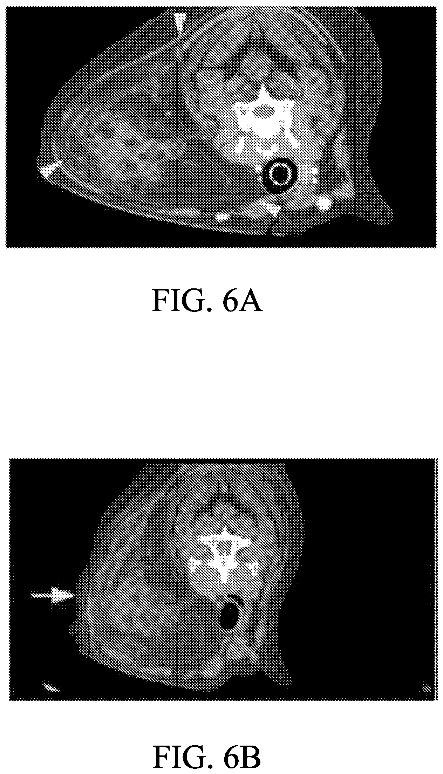

The results of computed tomography scan before the first administration are as follows: a huge, heterogeneous, fat-reduced subcutaneous lump (width: 12 cm, height: 9 cm, length: 17 cm) with central striped soft tissue was found, and the infiltration area thereof was horizontally from the right neck to the scapula (referring to FIG. 6A).

The results of computed tomography scan at conclusion visit are as follows: a huge, heterogeneous, fat-reduced subcutaneous lump (width: 12.60 cm, height: 10.64 cm, length: 16.08 cm) was found in the range from the horizontal to the right neck region of the scapula. The central striped soft tissue therein was suspected of necrosis and shrink, and the surrounding edges thereof were blurred (referring to FIG. 6 B).

According to the above results of the computed tomography scan, the volume of the lipoma almost unchanged. However, the area of necrosing and shrinking the striped soft tissue was increased, and blurred boundaries appeared around the edges thereof.

### Interim efficacy (appearance change):

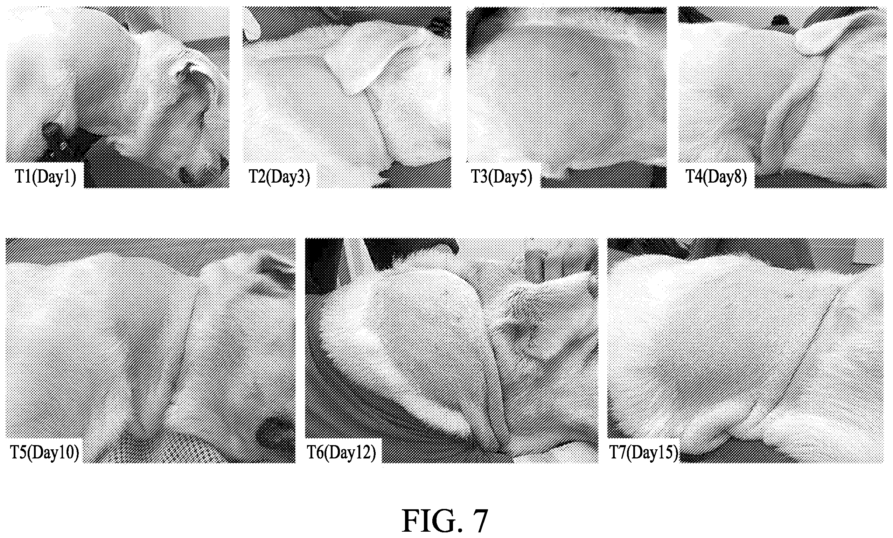

After 7 administrations of the GWA101 drug, the skin on the lipoma lesion of the dog had obvious changes, from being tight and unable to be pulled up to being loose and able to lift the skin in a large area (FIG. 7).

### Adverse effects:

No common side effects such as pain, nausea, vomiting or local redness and inflammation had been observed. The functions of liver and kidney were stable and normal during and after the treatment.

### Conclusion of this embodiment:

The pharmaceutical composition of the present disclosure can ablate fat and improve the quality of life and clinical symptoms of dogs suffering from lipomas. Furthermore, no significant increase in adverse reactions has been observed in the clinical trials.

## Claims

1. A composition comprising para-toluene sulfonamide and a pharmaceutically acceptable carrier for use in preventing or treating lipoma in a subject in need thereof, wherein the composition is to be administered to the subject in an effective amount.

2. The composition for use of claim 1, wherein the pharmaceutically acceptable carrier is selected from the group consisting of polyethylene glycol, alkylene glycol, propylene glycol, sebacic acid, dimethyl sulfoxide, ethanol, and any combination thereof.

3. The composition for use of claim 1, wherein the composition further comprises at least one of 10% to 40% by weight of polyethylene glycol, 5% to 10% by weight of propylene glycol, 1% to 5% by weight of sebacic acid, 0% to 15% by weight of *p*-Toluenesulfonic acid, 10% to 20% by weight of 2-ethyl-1,3-hexanediol, 0% to 10% by weight of dimethyl sulfoxide, and 0% to 20% by weight of anhydrous ethanol.

4. The composition for use of claim 1, wherein the para-toluene sulfonamide is present in an amount of from 1% to 60% by weight.

5. The composition for use of claim 1, wherein the composition is in a form selected from the group consisting of a formulation to injection, dry powder, a tablet, an oral liquid, a wafer, a film, a lozenge, a capsule, a granule, a pill, a gel, a lotion, an ointment, an emulsifier, a paste, and a cream.

6. The composition for use of claim 1, wherein the composition is for administration to the subject intratumorally, intravenously, subcutaneously, intradermally, intrathecally, intraperitoneally, intramuscularly, topically, orally, sublingually or intrapleurally.

7. The composition for use of claim 1, wherein the effective amount to be administered to the subject is from about 100 mg to about 26400 mg per day.

8. The composition for use of claim 1, wherein the effective amount to be administered to the subject is from about 300 mg to about 7000 mg per day.

9. The composition for use of claim 1, wherein the composition is for administration to the subject 1 to 4 times a day, 1 to 4 times a week, or 1 to 3 times a month.

## Patentansprüche

1. Zusammensetzung, umfassend *para*-Toluolsulfonamid und einen pharmazeutisch annehmbaren Träger, zur Verwendung bei der Vorbeugung oder Behandlung eines Lipoms bei einem Subjekt, das dessen bedarf, wobei die Zusammensetzung dem Subjekt in einer wirksamen Menge zu verabreichen ist.

2. Zusammensetzung zur Verwendung nach Anspruch 1, wobei der pharmazeutisch annehmbare Träger ausgewählt ist aus der Gruppe bestehend aus Polyethylenglykol, Alkylenglykol, Propylenglykol, Sebacinsäure, Dimethylsulfoxid, Ethanol und einer beliebigen Kombination davon.

3. Zusammensetzung zur Verwendung nach Anspruch 1, wobei die Zusammensetzung ferner mindestens eines von Folgendem umfasst: 10 Gew.-% bis 40 Gew.-% Polyethylenglykol, 5 Gew.-% bis 10 Gew.-% Propylenglykol, 1 Gew.-% bis 5 Gew.-% Sebacinsäure, 0 Gew.-% bis 15 Gew.-% *p*-Toluolsulfonsäure, 10 Gew.-% bis 20 Gew.-% 2-Ethyl-1,3-hexandiol, 0 Gew.-% bis 10 Gew.-% Dimethylsulfoxid und 0 Gew.-% bis 20 Gew.-% wasserfreies Ethanol.

4. Zusammensetzung zur Verwendung nach Anspruch 1, wobei das *para-*Toluolsulfonamid in einer Menge von 1 Gew.-% bis 60 Gew.-% vorliegt.

5. Zusammensetzung zur Verwendung nach Anspruch 1, wobei die Zusammensetzung in einer Form vorliegt, die ausgewählt ist aus der Gruppe bestehend aus einer Formulierung zur Injektion, einem Trockenpulver, einer Tablette, einer oralen Flüssigkeit, einer Oblate, einem Film, einer Lutschtablette, einer Kapsel, einem Granulat, einer Pille, einem Gel, einer Lotion, einer Salbe, einem Emulgator, einer Paste und einer Creme.

6. Zusammensetzung zur Verwendung nach Anspruch 1, wobei die Zusammensetzung zur intratumoralen, intravenösen, subkutanen, intradermalen, intrathekalen, intraperitonealen, intramuskulären, topischen, oralen, sublingualen oder intrapleuralen Verabreichung an das Subjekt bestimmt ist.

7. Zusammensetzung zur Verwendung nach Anspruch 1, wobei die dem Subjekt zu verabreichende wirksame Menge etwa 100 mg bis etwa 26400 mg pro Tag beträgt.

8. Zusammensetzung zur Verwendung nach Anspruch 1, wobei die dem Subjekt zu verabreichende wirksame Menge etwa 300 mg bis etwa 7000 mg pro Tag beträgt.

9. Zusammensetzung zur Verwendung nach Anspruch 1, wobei die Zusammensetzung zur Verabreichung an das Subjekt 1- bis 4-mal pro Tag, 1- bis 4-mal pro Woche oder 1-bis 3-mal pro Monat bestimmt ist.

## Revendications

1. Composition comprenant du *para*-toluènesulfonamide et un véhicule pharmaceutiquement acceptable, pour son utilisation dans la prévention ou le traitement d'un lipome chez un sujet en ayant besoin, dans laquelle la composition est destinée à être administrée au sujet en une quantité efficace.

2. Composition pour son utilisation selon la revendication 1, dans laquelle le véhicule pharmaceutiquement acceptable est choisi dans le groupe constitué par le polyéthylène glycol, l'alkylène glycol, le propylène glycol, l'acide sébacique, le diméthylsulfoxyde, l'éthanol et une quelconque combinaison de ceux-ci.

3. Composition pour son utilisation selon la revendication 1, dans laquelle la composition comprend en outre au moins l'un parmi 10 % à 40 % en poids de polyéthylène glycol, 5 % à 10 % en poids de propylène glycol, 1 % à 5 % en poids d'acide sébacique, 0 % à 15 % en poids d'acide *p*-toluènesulfonique, 10 % à 20 % en poids de 2-éthyl-1,3-hexanediol, 0 % à 10 % en poids de diméthylsulfoxyde et 0 % à 20 % en poids d'éthanol anhydre.

4. Composition pour son utilisation selon la revendication 1, dans laquelle le *para-*toluènesulfonamide est présent en une quantité de 1 % à 60 % en poids.

5. Composition pour son utilisation selon la revendication 1, dans laquelle la composition se présente sous une forme choisie dans le groupe constitué par une formulation pour injection, une poudre sèche, un comprimé, un liquide oral, une pastille, un film, une pastille à sucer, une capsule, un granulé, une pilule, un gel, une lotion, une pommade, un émulsifiant, une pâte et une crème.

6. Composition pour son utilisation selon la revendication 1, dans laquelle la composition est destinée à être administrée au sujet par voie intratumorale, intraveineuse, sous-cutanée, intradermique, intrathécale, intrapéritonéale, intramusculaire, topique, orale, sublinguale ou intrapleurale.

7. Composition pour son utilisation selon la revendication 1, dans laquelle la quantité efficace destinée à être administrée au sujet est d'environ 100 mg à environ 26400 mg par jour.

8. Composition pour son utilisation selon la revendication 1, dans laquelle la quantité efficace destinée à être administrée au sujet est d'environ 300 mg à environ 7000 mg par jour.

9. Composition pour son utilisation selon la revendication 1, dans laquelle la composition est destinée à être administrée au sujet 1 à 4 fois par jour, 1 à 4 fois par semaine, ou 1 à 3 fois par mois.
